# EUROPÄISCHE PATENTSCHRIFT

(11) **EP 1 532 213 B1**
(45) Veröffentlichungstag und Bekanntmachung des Hinweises auf die Patenterteilung: **11.03.2009**
(21) Anmeldenummer: 03797226.2
(22) Anmeldetag: 07.08.2003
(51) Int. Cl.: A61K 8/25, A61Q 1/08, A61Q 1/10, C09C 1/00, C09C 1/62, C09C 1/64, C09C 1/66

(54) **PIGMENT UND PIGMENTIERTES KOSMETISCHES PRAPARAT SOWIE VERFAHREN ZUR HERSTELLUNG DES PIGMENTS**
PIGMENT, PIGMENTED COSMETIC PREPARATION AND METHOD FOR PRODUCTION OF A PIGMENT
PIGMENT, PREPARATION COSMETIQUE PIGMENTEE ET PROCEDE DE FABRICATION DE CE PIGMENT

(30) Priorität: 21.08.2002 DE 10238090
(43) Veröffentlichungstag der Anmeldung: 25.05.2005
(73) Patentinhaber: Eckart GmbH, 90763 Fürth (DE)
(72) Erfinder: KAUPP, Günter, 91284 Neuhaus (DE); SCHUSTER, Thomas, 91207 Lauf (DE); KREMITZL, Hans-Jörg, 90542 Eckental (DE); SOMMER, Günter, 91217 Hersbruck (DE)
(74) Vertreter: LOUIS, PÖHLAU, LOHRENTZ
(86) Internationale Anmeldenummer: PCT/EP2003/008729
(87) Internationale Veröffentlichungsnummer: WO 2004/026268

(56) Entgegenhaltungen:
- EP-A- 0 338 428
- EP-A- 0 665 004
- WO-A-91/04293
- WO-A-95/14732
- DE-A- 19 836 810
- DATABASE WPI Section Ch, Week 199801 Derwent Publications Ltd., London, GB; Class D21, AN 1998-002739 XP0002265350 & JP 09 227114 A (MORI S) 2. September 1997 (1997-09-02)

## Beschreibung

Die Erfindung richtet sich auf ein Pigment für ein Kosmetikum, wie Lippenstift, Nagellack, Lidschatten, Haarfärbemittel, Mascara-Flüssigkeit, Selbstbräunungsflüssigkeit oder dergleichen und auf ein kosmetisches Präparat enthaltend ein derartiges Pigment.

Kosmetika der in Betracht stehenden Art, wie zum Beispiel lose oder gepresste Puder, Lidschatten, Lippenstifte, Eyeliner, Nagellacke, Rouges, Mascaras und dergleichen setzen sich zusammen aus einem Trägermaterial oder einer Basisformulierung sowie farb- und effektgebenden Mitteln verschiedener Art mit dem Ziel, auf Haut, Lippen oder Haaren einen bestimmten Farbeffekt zu erzielen.

Diese farb- und effektgebenden Mittel können Farbstoffe, verlackte organische Farbstoffe, anorganische oder organische Pigmente oder Effektpigmente sein, wobei insbesondere bei Effektpigmenten der Wunsch im Vordergrund steht, je nach dem Betrachtungswinkel des aufgebrachten Präparats einen unterschiedlichen Farbeindruck oder Helligkeitseindruck zu erzielen. Hierfür wurden im kosmetischen Bereich herkömmlicherweise insbesondere Perlglanzpigmente eingesetzt.

Perlglanzpigmente basieren auf plättchenförmigen Glimmerpartikeln als Substrat, die mit Metalloxiden, in erster Linie mit Titandioxid oder Eisenoxid beschichtet sind. Derartige Pigmente auf der Basis von Titanoxid sind jedoch aufgrund ihrer Zusammensetzung relativ transparent und zeigen in der Regel lediglich im sogenannten "Glanzwinkel" einen Farbeindruck, während auf Eisenoxid basierende Pigmente zwar deckender wirken, hier jedoch der winkelabhängige Farb- beziehungsweise Helligkeitseindruck in den Hintergrund gedrängt wird. Der Vorteil dieser Pigmente liegt in der hohen chemischen und thermischen Stabilität, die negative Einflüsse auf beispielsweise ein Bindemittel nahezu ausschließen, sowie in ihrer guten Hautverträglichkeit.

Soweit für kosmetische Zwecke bisher Metalleffektpigmente eingesetzt wurden, haben diese zwar den Vorteil, dass sie deckend, farbstark und hochbrillant sind, insbesondere weisen sie aber den Nachteil auf, dass sie den gesundheitlichen Anforderungen nicht gerecht werden, wobei vor allem zu berücksichtigen ist, dass Metall-Ionen aus dem Metall-Bestandteil, wie zum Beispiel Kupfer- oder Zink-Ionen, in das Trägermedium abgegeben werden und unerwünschte Effekte, wie Gelierung von Bindemitteln und Farbveränderungen, bewirken. Im applizierten Zustand kann es zu Kontakten mit Schweiß oder Speichel kommen, das heißt mit sauren oder basischen Medien, was ebenfalls eine verstärkte Freisetzung von Ionen bedingen kann, die nicht nur die Trägersubstanz beeinflussen, sondern unter Umständen auch unmittelbar zu gesundheitlichen Schäden, wie Hautirritationen, führen können.

Aus DE 44 37 753 A1 ist ein auch für kosmetische Zwecke einsetzbares Glanzpigment bekannt, welches aus mindestens fünf Schichten besteht und dementsprechend aufwändig in der Herstellung ist.

Die DE 198 36 810 A1 beschreibt Metallpigmente, die in einem wässrigen Medium beschichtet werden, was mit den nachstehend beschriebenen Nachteilen verbunden ist.

Die JP-09227114 betrifft mit Farbmitteln eingefärbte Perlglanzpigmente.

DE 101 14 445 A1 und DE 101 14 446 A1 beschreiben Eisenpigmente, die für kosmetische Anwendungen nicht zugelassen sind. Dies gilt entsprechend für das Eisenpigment gemäß EP 0 673 980 A2, welches bei erhöhter Temperatur in einer Sauerstoffatmosphäre behandelt wird.

US 6,398,861 B1 beschreibt eine Metallpigment-Zusammensetzung, nicht ein Metallpigment als solches. Es wird auf ein wässriges System hingewiesen sowie der Einsatz von Tensiden erwähnt, die für die im Folgenden beschriebene erfindungsgemäße Lösung völlig ungeeignet sind.

Hiervon ausgehend liegt der Erfindung die Aufgabe zugrunde, ein Pigment der eingangs genannten Art so auszugestalten, dass es den hygienischen und gesundheitlichen Anforderungen besser gerecht wird als herkömmlicherweise im kosmetischen Bereich eingesetzte Pigmente.

Diese Aufgabe wird erfindungsgemäß durch Bereitstellung eines Metallpigmentes gemäß Anspruch 1 gelöst.

Das erfindungsgemäß vorgesehene Sol-Gel-Verfahren beeinflusst wesentlich die Eigenschaft der das Substrat umkapselnden Schicht. Bei einem solchen Sol-Gel-Verfahren wird in organischer Lösung bzw. Suspension aus geeigneten monomeren Metalloxyd-Vorstufen, z. B. Alkoxysilane, unter Verwendung geeigneter Katalysatoren eine Barriereschicht um das metallische Substrat herum aufgebaut. Dieses Verfahren bietet gegenüber Beschichtungsverfahren aus wässrigen Lösungen, z. B. mit Wasserglas, den Vorteil, dass keine zusätzliche Vorbehandlung zur Aktivierung bzw.

Entfettung des mit Mahlhilfsmitteln belegten Basis-Pigments nötig ist und die erhaltene Schicht nicht durch weitere Ionen, wie z. B. Chloride oder Sulfate, kontaminiert werden kann. Zudem bietet eine so erhaltene Schicht, da sie aus monomeren Vorstufen erhalten wurde, den Vorteil einer besonders gleichmäßigen, dichten und somit hochwertigen optisch nicht wahrnehmbaren Schicht, die zudem auch aus gesundheitlichen und hygienischen Gesichtspunkten, wie sie insbesondere für die kosmetische Anwendung relevant sind, unbedenklich ist.

Ein derart ausgestaltetes Metallpigment weist keine oder eine deutlich verringerte Agglomerations- oder Flokkulationstendenz gegenüber einem nicht beschichteten oder im wässrigen System beschichteten Metallpigment auf. Die optischen Eigenschaften werden durch die umgebende Schicht nicht oder nicht wesentlich beeinflusst. Dies gilt entsprechend für die haptischen Eigenschaften.

Vorzugsweise ist die Schicht mit einem Bindemittel eines kosmetischen Präparats verträglich.

Die Schicht kann einerseits ein anorganisches Material enthalten oder hieraus bestehen, wobei dieses vorzugsweise ausgewählt wird aus der Gruppe Siliziumoxid, Titanoxid, Aluminiumoxid, Eisenoxid, Ceroxid und Chromoxid sowie Mischungen hieraus.

Andererseits beziehungsweise alternativ kann die Schicht organisches Material enthalten, welches günstigerweise ausgewählt wird aus der Gruppe Polyacrylate, Silicone, Polyolefine, Polystyrol, Polyester, Celluloseester, Polyamide, phosphororganische Substanzen, organisch modifizierte Silane, organisch modifizierte Titanate, organisch modifizierte Zirkonate sowie Mischungen hieraus.

Die Dicke der Schicht kann zwischen 5 und 500 nm, insbesondere zwischen 20 und 50 nm liegen.

Der metallische Kern besteht
aus Aluminium, wobei die Korngröße 100 % < 75 µm und 95 % < 45 µm ist und der Gehalt an Quecksilber ≤ 1 ppm, an Arsen ≤ 3 ppm, an Blei ≤ 20 ppm und der Al-Gehalt ≥ 99 % beträgt, oder
aus einem Bronzepigment dessen metallischer Kern einen Gehalt an Kupfer von 70 bis 95 %, einen Gehalt an Zink ≤ 30 % und einen Gehalt an Aluminium und Zinn von jeweils ≤ 0,5 % enthält sowie der Gehalt an Cadmium <= 15 ppm, an Blei <= 20 ppm, an Arsen <= 3 ppm und an Quecksilber <= 1 ppm beträgt, sowie die Korngröße zu 95 % < 45 µm ist, oder
aus einem Kupferpigment, dessen metallischer Kern einen Gehalt an Kupfer >= 95 % aufweist sowie der Gehalt an Cadmium <= 15 ppm, an Blei <= 20 ppm, an Arsen <= 3 ppm und an Quecksilber <= 1 ppm beträgt, sowie die Korngröße zu 95 % < 45 µm ist, oder
aus Silber besteht, wobei der Gehalt an Quecksilber <= 1 ppm, Arsen <= 5 ppm, Blei <= 10 ppm, sowie der Gehalt an Silber >= 99,9 % beträgt, oder der metallische Kern aus Gold besteht, wobei der Gehalt an Silber <= 7 %, Kupfer <= 4 %, sowie der Gehalt an Gold >= 90 % beträgt.

Es kann vorgesehen sein, dass der metallische Kern aus Aluminium besteht, der Gehalt an Quecksilber <=1 ppm, Arsen <= 3 ppm, Blei <= 10 ppm, Cadmium <= 1 ppm, Schwermetalle (als Blei) <= 40 ppm, Trocknungsverlust bei 105°C <= 0,5 %, sowie der Al-Gehalt >= 99 % beträgt.

Es kann günstigerweise eine Schicht derart vorgesehen sein, dass das Gewichtsverhältnis von Beschichtung zu metallischem Kern zwischen 1 und 0,001 liegt.

Ein erfindungsgemäßes Pigment kann sich weiterhin dadurch auszeichnen, dass der metallische Kern mittels eines pflanzlichen Schmiermittels, insbesondere pflanzlicher Ölsäure oder Stearinsäure, vermahlen ist und vorzugsweise plättchenförmig mit einem Durchmesser von 1 bis 100 µm und einer mittleren Dicke von 0,05 bis 2 µm ausgebildet ist.

Weiterhin betrifft die Erfindung ein Verfahren zur Herstellung eines Pigments, welches sich dadurch auszeichnet, dass die metallischen Substratteilchen ohne zusätzliche Vorbehandlung in einem Sol-Gel-Prozess in alkoholisch-wässriger Lösung durch Hydrolyse und Kondensation organischer Metalloxid-Vorstufen und ggf. unter Verwendung geeigneter Katalysatoren beschichtet werden.

Die Erfindung richtet sich auch auf ein kosmetisches Präparat enthaltend ein vorstehend charakterisiertes Pigment.

Nachfolgend wird die Erfindung anhand von zwei Beispielen zur Herstellung erfindungsgemäßer Metalleffektpigmente näher beschrieben:

### Beispiel 1:

In einem mit Rückflusskühler und Rührapparatur versehenen 1-Liter-Rundkolben werden 100 g eines mit pflanzlicher Stearinsäure vermahlenen Goldbronzepigmentes (mittlerer Teilchendurchmesser ca. 17 µm) in 500 ml Ethanol dispergiert, die Mischung wird auf 50° C erwärmt und mit 4,5 g einer 15 %-igen wässrigen Lösung von DMEA versetzt. Innerhalb von 8 Stunden wird dann eine Lösung von 17,3 g Tetraethoxysilan in 52 g Ethanol zudosiert. Nach beendeter Zugabe wird langsam abgekühlt und weitere 8 Stunden bei Raumtemperatur gerührt.

Das mit Si0₂ beschichtete Goldbronzepigment wird durch Filtration abgetrennt, mit 200 ml Ethanol nachgewaschen und bei 80 °C im Vakuumtrockenschrank getrocknet.

Das erhaltene Produkt hat einen SiO₂-Gehalt von 4,7 % und zeigt nach der Applikation in einem Nitrocellulose-Lack eine dem eingesetzten Ausgangsmaterial vergleichbare Optik mit hohem metallischem Glanz.

### Beispiel 2:

In einem mit Rückflusskühler und Rührapparatur versehenen 1-Liter-Rundkolben werden 75 g eines mit pflanzlicher Ölsäure vermahlenen Aluminiumpigmentes (mittlerer Teilchendurchmesser ca. 23 µm) in 600 ml Butylglykol dispergiert, die Mischung wird auf 95 °C erwärmt und mit 50 g einer 5 %-igen wässrigen Lösung von DMEA versetzt. Innerhalb von 12 Stunden wird dann eine Lösung von 24,7 g Tetraethoxysilan in 24,7 g Butylglykol zudosiert. Nach beendeter Zugabe wird langsam abgekühlt und weitere 8 Stunden bei Raumtemperatur gerührt.

Das mit Si0₂ beschichtete Aluminiumpigment wird durch Filtration abgetrennt, zwei mal mit je 200 ml Ethanol nachgewaschen und bei 100° C im Vakuumtrockenschrank getrocknet.

Das erhaltene Produkt hat einen Si0₂-Gehalt von 8,8 % und zeigt nach der Applikation in einem Nitrocellulose-Lack eine dem eingesetzten Ausgangsmaterial vergleichbare Optik mit hohem metallischem Glanz.

Nachfolgend werden zur besseren Veranschaulichung der Erfindung zwei Ausführungsbeispiele für kosmetische Präparate enthaltend erfindungsgemäße Pigmente beschrieben:

### Beispiel 1:

### Cremiges Augendekorationspräparat

| **Produktbezeichnung** | **Gew.-%** |
|---|---|
| Isopropylmyristat | 23 |
| Magnesiumstearat | 2 |
| Mineralöl | 25 |
| Bienenwachs | 40 |
| SiO₂-beschichtetes Goldbronzepigment | |
| (mittlere Partikelgröße: 35 µm) | 10 |

Die Fettmasse wird auf ca. 110° C erhitzt. Anschließend lässt man die Schmelze erkalten. Zu 2 g der Schmelze wird Pigment zugewogen. Es erfolgt dann ein erneutes vorsichtiges Schmelzen und Verrühren. Die so gewonnene nicht zu heiße Masse wird in eine Form gegossen.

### Beispiel 2:

### Rougepuder

| **Produktbezeichnung** | **Gew.-%** |
|---|---|
| Talcum | 33 |
| Kartoffelstärke | 20 |
| Magnesiumstearat | 8 |
| Calciumcarbonat | 4 |
| SiO₂-beschichtetes Goldbronzepigment | 19 |
| (mittlere Partikelgröße: 17 µm) | |
| SiO₂-beschichtetes Kupferpigment | 16 |
| (mittlere Partikelgröße: 17 µm) | |

Die Bestandteile werden gemischt und homogenisiert. Die Mischung wird bei 40 bar komprimiert und geformt.

## Patentansprüche

1. Metallpigment für ein kosmetisches Präparat, wie Lippenstift, Nagellack, Lidschatten, Haarfärbemittel, Mascara-Flüssigkeit, Puder, Eyeliner, Rouge, Haut/Haarpflegemittel, Parfum, Eau de Toilette, Lotions oder dergleichen, **dadurch gekennzeichnet, dass** ein metallisches Substrat eine im Sol-Gel-Verfahren hergestellte, eine Barrierewirkung gegenüber Schweiß und Speichel aufweisende, einen direkten Kontakt zwischen Haut und metallischem Substrat unterbindende, das Substrat umschließende Schicht aufweist, wobei
- der metallische Kern aus Aluminium besteht, die Korngröße 100 % < 75 µm und 95 % < 45 µm ist und der Gehalt an Quecksilber <= 1 ppm, Arsen <= 3 ppm, Blei <= 20 ppm, sowie der Al-Gehalt >= 99 % beträgt, oder
- der metallische Kern Kupfer umfasst und der Gehalt an Kupfer von 70 bis 95 %, einen Gehalt an Zink <= 30 % und einen Gehalt an Aluminium und Zinn jeweils <= 0,5 % enthält sowie der Gehalt an Cadmium <=15 ppm, an Blei <= 20 ppm, an Arsen <= 3 ppm und an Quecksilber <= 1 ppm beträgt, sowie die Korngröße zu 95 % < 45 µm ist, oder
- der metallische Kern im wesentlichen durch Kupfer gebildet ist, wobei der metallische Kern einen Gehalt an Kupfer >= 95 % aufweist sowie der Gehalt an Cadmium <=15 ppm, an Blei <= 20 ppm, an Arsen <= 3 ppm und an Quecksilber <= 1 ppm beträgt, sowie die Korngröße zu 95 % < 45 µm ist, oder
- der metallische Kern aus Silber besteht, der Gehalt an Quecksilber <= 1 ppm, Arsen <= 5 ppm, Blei <= 10 ppm, sowie der Gehalt an Silber >= 99,9 % beträgt, oder
- der metallische Kern aus Gold besteht, der Gehalt an Silber <= 7 %, Kupfer <= 4 %, sowie der Gehalt an Gold >= 90 % beträgt.

2. Metallpigment nach Anspruch 1, **dadurch gekennzeichnet, dass** die Schicht mit einem Bindemittel oder Träger des kosmetischen Präparats verträglich ist.

3. Metallpigment nach Anspruch 1 oder 2, **dadurch gekennzeichnet, dass** die Schicht anorganisches Material enthält oder hieraus besteht.

4. Pigment nach einem der Ansprüche 1 bis 3, **dadurch gekennzeichnet, dass** das anorganische Material aus der Gruppe Siliziumoxid, Titanoxid, Aluminiumoxid, Eisenoxid, Ceroxid und Chromoxid oder entsprechenden Hydraten sowie Mischungen hieraus ausgewählt wird.

5. Pigment nach einem der Ansprüche 1 bis 3, **dadurch gekennzeichnet, dass** die Beschichtung organisches Material enthält.

6. Pigment nach einem der Ansprüche 1 bis 3 oder 5, **dadurch gekennzeichnet, dass** das organische Material Polyacrylate, Silicone, Polyolefine, Polystyrole, Polyester, Celluloseester, Polyamide, phosphororganische Substanzen, organisch modifizierte Silane, organisch modifizierte Titanate, organisch modifizierte Zirkonate sowie Mischungen hieraus enthält.

7. Pigment nach einem der Ansprüche 1 bis 6 **dadurch gekennzeichnet, dass** die Schichtdicke der Umkapselung 5 bis 500 nm beträgt.

8. Pigment nach einem der Ansprüche 1 bis 7, **dadurch gekennzeichnet, dass** das Pigment mit einer Beschichtung versehen ist, wobei das Gewichtsverhältnis von Beschichtung zu metallischem Kern zwischen 1 und 0,001 liegt.

9. Pigment nach einem der Ansprüche 1 bis 8, **dadurch gekennzeichnet, dass** das metallische Substrat ein durch Vermahlung mit Schmiermitteln pflanzlichen Ursprungs hergestelltes Metallpigment ist.

10. Pigment nach einem der Ansprüche 1 bis 9, **dadurch gekennzeichnet, dass** der metallische Kern plättchenförmig mit einem Durchmesser von 1 bis 100 µm und einer mittleren Dicke von 0,05 bis 2 µm ausgebildet ist.

11. Verfahren zur Herstellung eines Pigments nach einem der vorstehenden Ansprüche, **dadurch gekennzeichnet, dass** die metallischen Substratteilchen ohne zusätzliche Vorbehandlung in einem Sol-Gel-Prozess in alkoholisch-wässriger Lösung durch Hydrolyse und Kondensation organischer Metalloxid-Vorstufen und gegebenenfalls unter Verwendung geeigneter Katalysatoren beschichtet werden.

12. Kosmetisches Präparat enthaltend ein Pigment nach einem der Ansprüche 1 bis 10.

## Claims

1. Metal pigment for a cosmetic preparation such as lipstick, nail varnish, eye shadow, hair colorant, mascara liquid, powder, eye liner, rouge, skin/hair care product, perfume, eau de toilette, lotions or the like, **characterised in that** a metallic substrate has a layer produced in the sol-gel process, having a barrier effect to perspiration and saliva, preventing a direct contact between skin and metallic substrate, surrounding the substrate, wherein
- the metallic core consists of aluminium, the particle size is 100% < 75 µm and 95% < 45 µm and the mercury content is <= 1 ppm, arsenic <= 3 ppm, lead <= 20 ppm and the Al content >= 99%, or
- the metallic core comprises copper and the copper content contains 70 to 95%, a zinc content <= 30% and an aluminium and tin content in each case <= 0.5% and the cadmium content is <= 15 ppm, lead <= 20 ppm, arsenic <= 3 ppm and mercury <= 1 ppm, and the particle size at 95% is < 45 µm, or
- the metallic core is formed substantially by copper, wherein the metallic core has a copper content of >= 95% and the cadmium content is <= 15 ppm, lead <= 20 ppm, arsenic <= 3 ppm and mercury <= 1 ppm, and the particle size at 95% is < 45 µm, or
- the metallic core consists of silver, the mercury content is <= 1 ppm, arsenic <= 5 ppm, lead <= 10 ppm and the silver content >= 99.9%, or
- the metallic core consists of gold, the silver content is <= 7%, copper <= 4% and the gold content >= 90%.

2. Metal pigment according to Claim 1, **characterised in that** the layer is compatible with a binding agent or carrier of the cosmetic preparation.

3. Metal pigment according to Claim 1 or 2, **characterised in that** the layer contains inorganic material or consists thereof.

4. Pigment according to one of Claims 1 to 3, **characterised in that** the inorganic material is selected from the group silicon oxide, titanium oxide, aluminium oxide, iron oxide, cerium oxide and chromium oxide or corresponding hydrates and mixtures thereof.

5. Pigment according to one of Claims 1 to 3, **characterised in that** the coating contains organic material.

6. Pigment according to one of Claims 1 to 3 or 5, **characterised in that** the organic material contains polyacrylates, silicones, polyolefins, polystyrenes, polyesters, cellulose esters, polyamides, phosphororganic substances, organically modified silanes, organically modified titanates, organically modified zirconates and mixtures thereof.

7. Pigment according to one of Claims 1 to 6, **characterised in that** the layer thickness of the encapsulation is 5 to 500 nm.

8. Pigment according to one of Claims 1 to 7, **characterised in that** the pigment is provided with a coating, wherein the proportion by weight of coating to metallic core is between 1 and 0.001.

9. Pigment according to one of Claims 1 to 8, **characterised in that** the metallic substrate is a metal pigment produced by grinding with lubricants of plant origin.

10. Pigment according to one of Claims 1 to 9, **characterised in that** the metallic core is formed platelet-like with a diameter of 1 to 100 µm and a mean thickness of 0.05 to 2 µm.

11. Process for the production of a pigment according to one of the preceding claims, **characterised in that** the metallic substrate particles are coated without additional pre-treatment in a sol-gel process in alcoholic-aqueous solution by hydrolysis and condensation of organic metal oxide precursors and optionally using suitable catalysts.

12. Cosmetic preparation containing a pigment according to one of Claims 1 to 10.

## Revendications

1. Pigment métallique pour une préparation cosmétique, comme du rouge à lèvres, du vernis à ongles, du fard à paupières, des teintures pour les cheveux, du liquide de mascara, de l'Eyeliner, du fard à joue, des produits de soins de la peau/des cheveux, du parfum, de l'eau de toilette, des lotions ou des produits similaires, **caractérisé en ce qu'**un substrat métallique présente une couche fabriquée dans le cadre du procédé sol-gel, présentant une action barrière contre la sueur et la salive, empêchant un contact direct entre la peau et le substrat métallique, entourant le substrat,
- l'âme métallique se composant d'aluminium, la granulométrie étant pour 100 % < 75 µm et pour 95 % < 45 µm et la teneur en mercure étant <= 1 ppm, en arsenic <= 3 ppm, en plomb <= 20 ppm, ainsi que la teneur en Al étant >= 99 % ou
- l'âme métallique comprenant du cuivre et la teneur en cuivre étant de 70 à 95 %, la teneur en zinc <=30 % et la teneur en aluminium et en étain étant à chaque fois <= 0,5 % ainsi que la teneur en cadmium étant <= 15 ppm, en plomb <= 20 ppm, en arsenic <= 3 ppm et en mercure <= 1 ppm, ainsi que la granulométrie étant pour 95 % < 45 µm ou
- l'âme métallique étant formée pour l'essentiel par du cuivre, l'âme métallique présentant une teneur en cuivre >= 95 % ainsi qu'une teneur en cadmium <= 15 ppm, en plomb <= 20 ppm, en arsenic <= 3 ppm et en mercure <= 1 ppm, ainsi qu'une granulométrie pour 95 % < 45 µm ou
- l'âme métallique se composant d'argent, la teneur en mercure étant <= 1 ppm, en arsenic <= 5 ppm, en plomb <= 10 ppm ainsi que la teneur en argent étant >= 99,9 % ou
- l'âme métallique se composant d'or, la teneur en argent étant <= 7 %, en cuivre <= 4 % ainsi que la teneur en or étant >= 90 %.

2. Pigment métallique selon la revendication 1, **caractérisé en ce que** la couche est compatible avec un liant ou un excipient/vecteur de la préparation cosmétique.

3. Pigment métallique selon la revendication 1 ou 2, **caractérisé en ce que** la couche contient ou se compose d'un matériau inorganique.

4. Pigment selon l'une quelconque des revendications 1 à 3, **caractérisé en ce que** le matériau inorganique est sélectionné parmi le groupe oxyde de silicium, oxyde de titane, oxyde d'aluminium, oxyde de fer, oxyde de cérium et oxyde de chrome ou parmi leurs hydrates correspondants ainsi que parmi des mélanges de ces derniers.

5. Pigment selon l'une quelconque des revendications 1 à 3, **caractérisé en ce que** le revêtement contient un matériau organique.

6. Pigment selon l'une quelconque des revendications 1 à 3 ou 5, **caractérisé en ce que** le matériau organique contient des polyacrylates, des silicones, des polyoléfines, des polystyrènes, des polyesters, des esters de cellulose, des polyamides, des substances organo phosphoriques, des silanes modifiés par voie organique, des titanates modifiés par voie organique, des zirconates modifiés par voie organique ainsi que des mélanges de ces derniers.

7. Pigment selon l'une quelconque des revendications 1 à 6, **caractérisé en ce que** l'épaisseur de couche de l'encapsulation est de 5 à 500 nm.

8. Pigment selon l'une quelconque des revendications 1 à 7, **caractérisé en ce que** le pigment est pourvu d'un revêtement, le rapport pondéral du revêtement à l'âme métallique étant compris entre 1 et 0,001.

9. Pigment selon l'une quelconque des revendications 1 à 8, **caractérisé en ce que** le substrat métallique est un pigment métallique préparé par mouture avec des graisses d'origine végétale.

10. Pigment selon l'une quelconque des revendications 1 à 9, **caractérisé en ce que** l'âme métallique est formée sous forme de plaquettes ayant un diamètre de 1 à 100 µm et une épaisseur moyenne de 0,05 à 2 µm.

11. Procédé en vue de la fabrication d'un pigment selon l'une quelconque des revendications précédentes, **caractérisé en ce que** les particules de substrat métalliques sont revêtues, sans traitement préalable supplémentaire, dans un processus sol-gel en solution alcoolique-aqueuse par hydrolyse et condensation de précurseurs d'oxydes métalliques organiques et, le cas échéant, en utilisant des catalyseurs appropriés.

12. Préparation cosmétique contenant un pigment selon l'une quelconque des revendications 1 à 10.
